# EUROPEAN PATENT APPLICATION

(11) **EP 0 974 599 A1**
(43) Date of publication of application: **26.01.2000**
(21) Application number: 97912426.0
(22) Date of filing: 12.11.1997
(51) Int. Cl.: C07K 14/59

(54) **RECOMBINANT SINGLE-STRANDED EQUINE CHORIONIC GONADOTROPIN**

(30) Priority: 12.11.1996 JP 30004196
(71) Applicant: TEIKOKU HORMONE MFG. CO., LTD., Tokyo 107 (JP)
(72) Inventor: SHIOTA, Kunio, Inba-gun, Chiba 270-1423 (JP); OGAWA, Tomoya, Musashino-shi, Tokyo 180-0001 (JP); MIN, Kwan-Sik, Iowa City, IA 52246 (US)
(74) Representative: Weisert, Annekäte, Dipl.-Ing. Dr.-Ing.
(86) International application number: JP9704113
(87) International publication number: WO9821238

(57) **Abstract**

A novel recombinant single-stranded equine chorionic gonadotropin (eCG) having the amino acid sequence of SEQ ID NO: 5, comprising an α chain and a β chain of eCG connected to each other. It has about seven times as high FSH-like activity as naturally occurring and wild type eCGs.

## Description

### Technical field

The present invention relates to a recombinant single chain equine chorionic gonadotropin (eCG). A recombinant single chain eCG in which an α chain and a β chain of the eCG are tethered to a single chain has potent FSH activity about 7 times than that of the native eCG, and can be used advantageously as a medicine for animals.

### Background art

Native chorionic gonadotropin is a member of the glycoprotein hormone family and is constituted by an α-subunit noncovalently linked to a β-subunit as in luteinizing hormone (LH) or follicle-stimulating hormone (FSH) derived from pituitary. An equine chorionic gonadotropin (eCG) has both of the LH activity and FSH activity, has common primary structures of the peptides to eLH and their amino acid sequences are identical. However, their sugar chain structures are different from each other and thus eCG is extremely unique among the glycoprotein hormone family.

That is, an amino acid sequence and a nucleic acid sequence of the α-subunit of eCG are as shown in SEQ.ID. NO:1 and SEQ.ID. NO:2, respectively, and comprise 96 amino acids having signal peptides of 24 amino acids (J. Repred. Dev. 40, 301 (1994)). And two asparagines at the 56th and the 82nd of the α-subunit are both N-bond glycosylated. Incidentally, in the SEQ.ID. NO:1. -24 to -1 are signal peptides and are not contained in the subunits of mature.

Also, an amino acid sequence and a nucleic acid sequence of the β-subunit of eCG are as shown in SEQ.ID. NO:3 and SEQ.ID. NO:4, respectively, and the β-subunit eCG comprises 149 amino acids having signal peptides of 20 amino acids (Mol. Endocrinol. 6, 951 (1992)). And asparagine at the 13th thereof is N-bond glycosylated, and the OH groups of Ser of 4 to 11 at the molecule C-terminus side are glycosylated (Fed. Proc. 45, 1843 (1986), Fed. Proc. 45, 188 (1986), J. Biol. Chem. 262, 8683 (1987), Ann. Meet Endocrinol. (Abstract) 1200 (1992)). Incidentally, in the SEQ.ID. NO:3, -20 to -1 are signal peptides and are not contained in the subunit of mature.

### Disclosure of the invention

The present invention comprises a recombinant single chain eCG represented by the amino acid sequence shown in SEQ. ID. NO:5, in which an α-chain (an α-subunit) and a β-chain (a β-subunit) of equine chorionic gonadotropin (eCG) are tethered to a single chain.

The present invention is also to provide a DNA sequence represented by SEQ.ID. NO:6 which encodes the recombinant single chain eCG.

In the present invention, a veterinary drug containing the above-mentioned recombinant single chain eCG can be further provided.

### Brief description of the drawings

Fig. 1 is an outlined view showing a constructing method of a recombinant single chain eCG by the overlapping PCR.
Fig. 2 is an outlined view showing construction of an expression transfer vector, pAB-tethered eCG α/β.
Fig. 3 is to show an LH-like activity of the recombinant single chain eCG of the present invention.
Fig. 4 is to show an FSH-like activity of the recombinant single chain eCG of the present invention.

### Best mode for practicing the invention

The recombinant single chain eCG has substantially the same biological activities, particularly in a luteinizing hormone (LH)-like activity as compared with the native eCG and the wild-type eCG (Endocrine Journal 1996, 43(5) 585-593) synthesized by two chains, but the inventors have found that it has a follicle-stimulating hormone (FSH)-like activity about 7 times more potent than those of the above. Accordingly, the present invention is to provide a novel recombinant single chain eCG which can advantageously express hormones in expression cell series such as CHO-K1 cells.

The amino acid sequence of the recombinant single chain eCG according to the present invention is as shown in SEQ.ID. NO:5, and the amino acid sequence from the 1st to the 149th corresponds to a β-subunit, a sugar chain binds to Asn at the 13th, and a sugar chain (OCHO) also binds to seven Ser's. The amino acid sequence from the 150th to the 245th corresponds to an α-subunit, and sugar chains bind to Asn's at the 205th and the 231th. Incidentally, in SEQ.ID. NO:5, -20 to -5 are signal peptides and are not contained in the single chain eCG of mature. The DNA sequence which encodes the recombinant single chain eCG of the present invention is shown by SEQ.ID. NO:6. The base sequences from 504th to the 530th are an anti-sense of Primer 2, and the base sequences from the 507th to the 533th are derived from Primer 3.

In the present invention, the above-mentioned recombinant single chain eCG can be used as a veterinary drug. This veterinary drug can be applied to laboratory animals selected from rats, mice and rabbits, or animals such as domestic animals, etc. selected from bovines, horses, pigs and sheeps. Also, the veterinary drug is effective for superovulation, folliclar cyst, ovulation failure, ovarian subfunction, induction of postpartum estrus or hypoorehidia by administering its effective dose.

### Examples

The recombinant single chain eCG of the present invention can be produced as mentioned below.

### (1) Construction of tethered eCG transfer vector

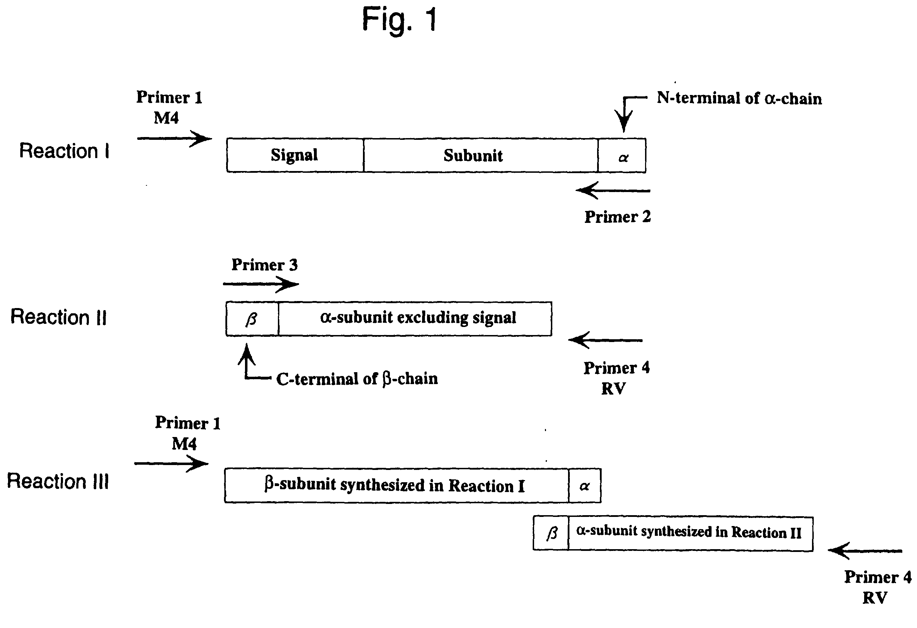

Nucleic acid sequence which encodes eCG α-subunit is inserted into the 3' terminal frame of the eCG β-subunit by using the overlapping PCR mutangenesis (Fig. 1).

The following primers were used in the construction.
Primer 1, M13 forward (M4), 5'-GTTTTCCCAGTCACGAC-3' (SEQ.ID. NO:7);
Primer 2, 5'-TCCATCAGGAAAAGAAGTCTTTATTGG-3' (SEQ.ID. NO:8);
Primer 3, 5'-ATAAAGACTTCTTTTCCTGATGGAGAG-3';
Primer 4, M13 reverse (RV), 5'-CAGGAAACAGCTATGAC-3' (SEQ.ID. NO:9).

PCR was carried out using previously cloning eCG α-subunit and β-subunit cDNA as templates.

The first reaction I was carried out using Primers 1 and 2. Primer 2 contains the first four codons of the mature α-subunit and the five C-terminal codons of the eCG β-subunit.

In the second reaction II, Primer 3 was used. Primer 3 contains the sequence corresponding to the last four C-terminal codons of the eCG β-subunit and the first five codons of the matured α-subunit.

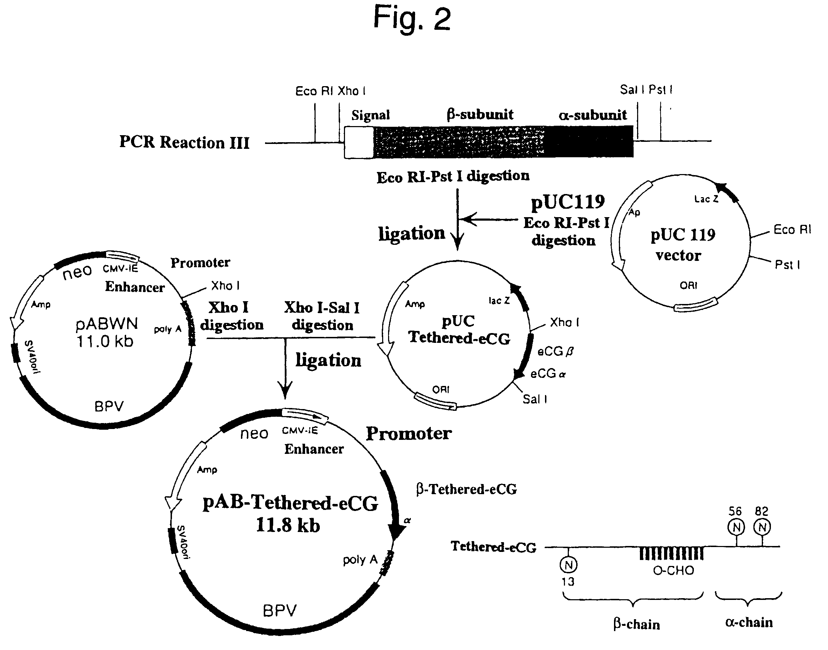

The fragments of the reactions I and II were annealed, and PCR of the final reaction III was carried out using primers 1 and 4 so as to generate the tethered single chain eCG. This fragment was digested as shown in Fig. 2 with *Eco* RI/*Pst* I, ligated into the same sites of pUC119 vector (pUC Tethered-eCG). To ensure that no errors had been introduced during the PCR, the sequence was determined.

With regard to the recombinant single chain eCG of the present invention, a nucleic acid sequence encoding a sufficient length eCG β-subunit (20-amino acid residue signal sequence and 149 amino acid residues of the matured protein) was fused with the 5'-end of the eCG α-subunit cDNA that includes the coding sequence for the mature protein, but not the signal sequence. Following Xho I/Sal I digestion (834 bp), the latter fragment was ligated into the unique Xho I site of the pABWN expression vector to be transfected into CHO-K1 cells. This expression vector was named as a pAB-tethered single chain eCG. The direction of the expression transfer vector was confirmed by restriction mapping.

### (2) Cell culture

The expression vector was gene transfected into CHO-K1 cells by the liposome formulation (Lipofectamine) according to the supplier's instructions. Stable clones were selected by incubation in growth medium (Ham's F12 medium containing penicillin (50 units/ml), streptomycin (50 µg/ml), glutamine (2 mM) and 10% FCS) supplemented with G418 (800 µg/ml) in the presence of 5% CO₂ + 95% air at 37°C for 2 weeks after gene transfection.

### (3) PCR and Northern blotting analysis of prepared stable cells

The supernatant of the selected cells was removed and the cells were treated with trypsin in a 125 cm² of tissue culture flask. The cells were collected and the whole RNAs were prepared by using Chomczynski's extracting method (1993).

PCR was carried out to determine an expressing recombinant single chain eCG. 20 µg of RNA was used for the Northern blotting analysis. The eCG α-subunit and β-subunit cDNAs labeled by the random primer method as well as hybridization were used.

### (4) Expression of recombinant single chain eCG

After incubation of selected stable cells (1 × 10⁶) in 20 ml CHO-S-SFM-II containing penicillin (50 units/ml) and streptomycin (50 µg/ml) at 37°C for 48 hours, the culture supernatant were collected and centrifuged at 100,000 × g, 4°C for 60 min to remove the cell debris.

The recombinant single chain eCG expressed in the supernatant was quantified using RIA according to the supplier's protocol (UCB-Bioproducts) of an anti-eCG antibody.

### (5) Quantification of recombinant single chain eCG by radioimmunoassay

Known wild type eCG and the recombinant single chain eCG of the present invention were quantified using a polyclonal antibody (A540/R1H) by eCG radioimmunoassay. To each tube were added 200 µl of 0.02M phosphate buffer (0.017M Na₂HPO₄. 12H₂O, 0.003M KH₂PO₄, 0.9% (w/v) NaCl, 0.5% (w/v) BSA, 0.02% (w/v) NaN₃), 100 µl of native eCG standard (6.25, 12.5, 25, 50, 100, 200 or 400 ng), 100 µl of rabbit anti-eCG polyclonal antibody (7 µg/ml) and 100 µl of ¹²⁵I eCG tracer (20,000 cpm/100 µl) labeled by the chloramine T method (Katayama et al, 1990). After incubation at 4°C overnight, 500 µl of anti-rabbit precipitating antibody was added to the respective tubes and each mixture was incubated for 30 minutes. Then, 2 ml of the phosphate buffer was added to each mixture, each mixture was centrifuged at 3,000 rpm for 15 minutes, the supernatant was suctioned and a count was measured.

### (6) Bioassay for LH-like activity using Leydig cells

The LH-like activities of the wild type eCG and the recombinant single chain eCG were determined using a rat Leydig cell *in vitro* culture system. Testes of four 60-days-old Sparague Dawley (SD) rats were cut off and decapsulated carefully. The cells were dispersed in a 199 medium containing collagenase (0.25 mg/ml) and BSA (1 mg/ml) while shaking under 150 cycles/min at 34°C for 30 minutes. After completing tubule dispersion, the tubes were stood vertically to sediment the tubule fraction at room temperature for 5 minutes. The supernatants were collected and filtered through a gauze made of Nylon, and centrifuged at 600 g for 15 minutes, and the sedimented interstitial cells were resuspended in a 199 medium.

The bioassay of the wild type eCG and the recombinant single chain eCG was carried out using 24-well plates. Each well contained 0.5 ml of a medium containing 1 x 10⁶ cells in a suspended state and 0.1 ml of the native eCG (2 to 128 ng) or a recombinant sample (2 to 128 ng) to be determined by RIA. The plates were incubated at 37°C for 4 hours while the plates were subjected to continuous shaking, then centrifuged at 2,500 rpm for 10 minutes, and the testosterone in the culture supernatant was measured by RIA mentioned below.

### (7) Bioassay for FSH-like activity using granulosa cells

This *in vitro* bioassay was performed based on measurement of the amount of estradiol converted from 19-hydroxy-androstene-dione by FSH induced aromatase activity in granulosa cells of 21 to 22-days-old rats. That is, a capsule (10 mm) made of a silicon containing about 10 mg of DES was implanted in SD rats to promote growth of the granulosa cells. Four days later, animals were sacrificed by cervical dislocation, ovaries were cut open and capsules were taken out. The ovarian follicles were punctured with a 27-gauge hypodermic needle and the granulosa cells were carefully expressed in a McCoy's 5a medium. These cells were centrifuged at 800 rpm for 5 minutes, the supernatant was removed, washed with a fresh McCoy's 5a medium and centrifuged. These cells were suspended again in the medium to produce a final concentration of 50,000 to 80,000 viable cells/60 µl. To each well of 24-well plates was added 400 µl of a GAB assay medium (100 U/ml of penicillin, 100 µg/ml of streptomycin, 2 mM of L-glutamine, 1 µM of androstene-dione, 10⁻⁷ M of DES, 30 ng/ml of hCG, 1 µg/µl of insulin and 0.125 mM MIX in McCoy's 5a medium), and then, to the well were added 40 µl of a sample (2 to 64 ng of native eCG or 2 to 64 ng of a recombinant sample to be determined by RIA) and 60 µl of the cell suspension. These cells were cultured in an incubator under an atmosphere of 95% air + 5% CO2 at 37°C for 3 days, then these plates were removed from the incubator and centrifuged at 1,000 rpm for 3 minutes, and the estradiol concentrations in the culture supernatant were measured by RIA mentioned below.

### (8) RIA of testosterone and estradiol concentrations

Concentrations of testosterone and estradiol secreted into culture supernatant were measured by RIA using the culture supernatant. The culture supernatant was extracted twice with diethyl ether, the extract was diluted with 1 ml of acetone, and the mixture was divided into two portions. Each portion was placed in a tube, 500 µl of an assay buffer, (0.1 M phosphate buffer (pH 7.0), 0.9% (w/v) NaCl, 0.05% (w/v) NaN₃, 0.1% (w/v) gelatin), 100 µl of standard testosterone or estradiol (50, 100, 200, 400, 800 or 1,600 pg), 100 µl of anti-testosterone (diluted 1:1,500) or anti-estradiol (diluted 1:4,500) antiserum and 100 µl of ³H-testosterone or ³H-estradiol (10,000 cpm) were added. After incubating at 4°C overnight, 200 µl of an activated charcoal liquid (0.625% (w/v) Norita A and 0.0625% (w/v) dextrane T-70 in the assay buffer) was added and incubation was carried out at 4°C for 20 minutes. The cells were centrifuged at 3,000 rpm and 4°C for 20 minutes, and the supernatant was decanted into vials, then, 3 ml of scintillator (4.2% (v/v) Liquifluor TM/toluene) was added thereto and radioactivity was measured by using a liquid scintillation counter.

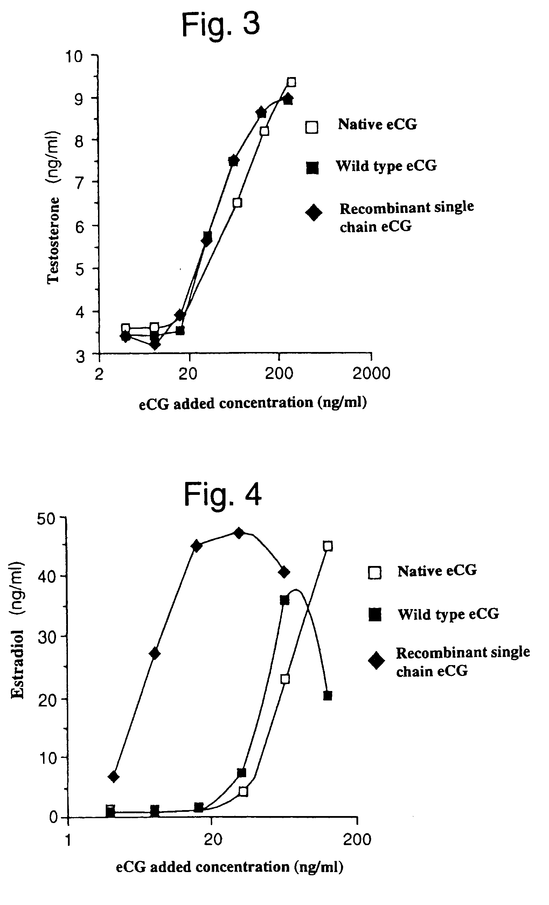

With regard to the LH-like activity, the recombinant single chain eCG of the present invention was extremely similar to that of the wild type eCG (Fig. 3), but with regard to the FSH-like activity, the recombinant single chain eCG of the present invention had potent activity about 7 times than that of the wild type eCG (Fig. 4).

Incidentally, relative promotion activity obtained from EC₅₀ values is shown in Table 1.

**Table 1**

| Hormone expression in CHO-K1 cells | | | |
|---|---|---|---|
| | Hormone expression (ng/100 µl) | *In vitro* activity (%) | |
| | | LH | FSH |
| Recombinant single chain-eCG | 201 ± 22 | 100 | 684.9 |
| Wild type eCG | 208 ± 29 | 100 | 100 |

### Utilizability in industry

The recombinant single chain eCG of the present invention can be used as a veterinary drug for animals such as laboratory animals selected from rats, mice and rabbits, or animals such as domestic animals, etc. selected from bovines, horses, pigs and sheeps. This veterinary drug is effective for super-ovulation, folliclar cyst, ovulation failure, ovarian subfunction, induction of postpartum estrus or hypoorchidia.

## Claims

1. A recombinant single chain eCG represented by the amino acid sequence shown in SEQ.ID. NO:5 in which an α-chain and a β-chain of equine chorionic gonadotropin (eCG) are tethered to a single chain.

2. A DNA sequence represented by SEQ.ID. NO:6 encoding the recombinant single chain eCG according to Claim 1.

3. A veterinary drug containing the recombinant single chain eCG according to Claim 1.

4. The veterinary drug according to Claim 3, wherein the animals to be applied to is laboratory animals selected from rats, mice and rabbits, or domestic animals selected from bovines, horses, pigs and sheeps.

5. The veterinary drug according to Claim 3, which drug is used superovulation, folliclar cyst, ovulation failure, ovarian subfunction, induction of postpartum estrus or hypoorchidia.
